# EUROPEAN PATENT SPECIFICATION

(11) **EP 1 018 513 B1**
(45) Date of publication and mention of the grant of the patent: **01.02.2006**
(21) Application number: 99123695.1
(22) Date of filing: 18.05.1995
(51) Int. Cl.: C07D 451/06

(54) **8-(2,2,2-Trifluoroethyl)-8-azabicyclo[3.2.1]octan-3-one useful as intermediate for serotonin 4 receptor stimulating quinolinecarboxylic acid derivatives**
8-(2,2,2-Trifluoroethyl)-8-azabicyclo[3.2.1]octan-3-on verwendbar als Zwischenprodukt für Serotonin-4-Rezeptor stimulierende Chinolincarbonsäure-Derivate
8-(2,2,2-Trifluoroéthyl)-8-azabicyclo[3.2.1]octan-3-one utile comme intermédiaire pour dérivés d'acide quinolinecarboxylique stimulant le récepteur sérotonine 4

(30) Priority: 18.05.1994 JP 10317794
(43) Date of publication of application: 12.07.2000
(62) Divisional of application: 95918742.8
(73) Proprietor: Syngenta Limited, Guildford Surrey GU2 7YH (GB)
(72) Inventor: Ohuchi, Yutaka, Toshima-ku, Tokyo 171 (JP); Suzuki, Masaji, Toshima-ku, Tokyo 171 (JP); Asanuma, Hajime, Toshima-ku, Tokyo 171 (JP); Yokomori, Sadakazu, Toshima-ku, Tokyo 171 (JP); Hatayama, Katsuo, Toshima-ku, Tokyo 171 (JP)
(74) Representative: Waterman, John Richard

(56) References cited:
- MARIA L. IZQUIERDO ET AL.: "Synthesis and structural, conformational and pharmacological study of N-beta(or gamma)acyloxyalkylnortropinones" EUROPEAN JOURNAL OF MEDICINAL CHEMISTRY., vol. 24, no. 2, 1989, pages 123-129, XP002252544 EDITIONS SCIENTIFIQUE ELSEVIER, PARIS., FR ISSN: 0223-5234
- CHEMICAL ABSTRACTS, vol. 109, no. 3, 18 July 1988 (1988-07-18), Columbus, Ohio, US; abstract no.: 22817t, GUTKOWSKA, BOZENNA ET AL.: "Synthesis of some amide derivatives of 3-amino-tropane with expected pharmaclogical activity.II." XP002252545 & ACTA POL. PHARM., vol. 43, no. 6, 1986, pages 553-558, -& DATABASE CAPLUS CHEMICAL ABSTRACTS SERVICE, COLUMBUS, OHIO, US; CA 109:22817 XP002252547
- CHEMICAL ABSTRACTS, vol. 106, no. 23, 8 June 1987 (1987-06-08), Columbus, Ohio, US; abstract no.: 195780s, ARIAS, M.S. ET AL.: "Conformational study of N-substituted 8-azabicyclo[3.2.1]octan-3-ones" XP002252546 & J. MOL: STRUCT:, vol. 147, no. 3-4, 1986, pages 381-388, -& DATABASE CAPLUS CHEMICAL ABSTRACTS SERVICE, COLUMBUS, OHIO, US; CA 106:195780 XP002252548

## Description

The present invention relates to a tropinone compound useful as an intermediate for quinolinecarboxylic acid derivatives which have an action for stimulating a serotonin 4 receptor.

Derivatives of N-alkyl tropinones are known from Eur. J. Med. Chem (1989), vol.24, no. 2., p123-129, Chemical Abstracts (1988), vol.109, no.3, abstract no.22817t and J. Mol. Struct. (1986), vol. 147, no.3-4, p381-388.

According to the present invention there is provided the compound 8-(2,2,2-trifluoroethyl)-8-azabicyclo[3.2.1]octan-3-one.

The compound is useful as an intermediate for quinolinecarboxylic acid derivatives represented by formula (A) 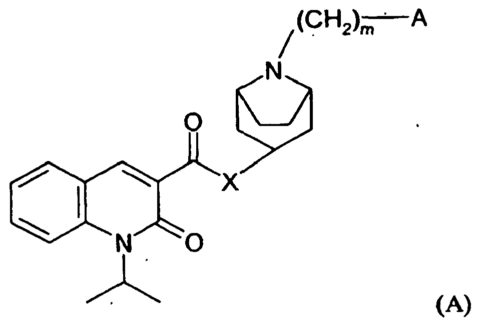
wherein
X represents an oxygen atom or imino group;
m represents 0 or an integer of 1 to 6; and,
A represents an alkenyl group, an alkynyl group, a haloalkyl group, hydroxy group, an alkoxy group, an acyloxy group, an alkoxyalkoxy group, a mono-or di-alkylamino group, an alkylthio group, an alkyl-sulfinyl group, an alkylsulfonyl group, an arylsulfonyl group, an aryloxy group, morpholinyl group, piperidyl group, tetrahydropyranyl group, an alkoxycarbonyl group, carboxyl group, an alkanoyl group, cyano group or carbamoyl group; or a pharmaceutically acceptable salt thereof.
8-(2,2,2-Trifluoroethyl)-8-azabicyclo[3.2.1]octan-3-one may be prepared by reacting H₂NCH₂CF₃ with butanedial and 1,3-aceionedicarboxylic acid or its ester, by a modification of the method disclosed in Journal of Organic Chemistry, 22, 1385 (1957).

That is, butanedial obtained by treating 2,5-dimethoxytetrahydrofuran with hydrochloric acid is reacted with H₂NCH₂CF₃ and 1,3-acetonedicarboxylic acid in a solvent to give 8-(2,2,2-Trifluoroethyl)-8-azabicyclo[3.2.1]octan-3-one. Examples of the solvent used for the reaction are a hydrated solvent of ethanol, methanol, N,N-dimethylformamide or dimethylsulfoxide; and water. The reaction temperature is appropriately chosen from the range of -20°C to the boiling point of the solvent. In general, the reaction time is appropriately between 30 minutes and 48 hours. It is preferred to adjust the reaction solution to an acidic region. Preferably the reaction is carried out by adjusting the pH to approximately 1.5 to 4.5, using, e.g., hydrochloric acid, sodium hydrogenphosphate or sodium hydroxide.

### Example

8-(2,2,2-Trifluoroethyl)-8-azabicyclo[3.2.1]octan-3-one was prepared as outlined above.
MS (m/z); 207, 150
NMR (ppm, CDCl3);
1.66 (2H, d, J=7.8Hz), 1.95-2.15 (2H, m), 2.24 (2H, dd, J=17.2, 1.6Hz), 2.68 (2H, dd, J=16.4, 4.6Hz), 3.15 (2H, q, J=9.2Hz), 3.50-3.70 (2H, m)

## Claims

1. 8-(2,2,2-Trifluoroethyl)-8-azabicyclo[3.2.1]octan-3-one.

## Patentansprüche

1. 8-(2,2,2-Trifluorethyl)-8-azabicyclo[3.2.1]octan-3-on.

## Revendications

1. 8-(2,2,2-trifluoroéthyl)-8-azabicyclo[3.2.1]octan-3-one.
